(19) Europäisches Patentamt
European Patent Office
Office européen des brevets

(11) Publication number : **0 404 322 B1**

# (12) EUROPEAN PATENT SPECIFICATION

(45) Date of publication of patent specification :
**22.09.93 Bulletin 93/38**

(51) Int. Cl.$^5$ : **C07D 239/95,** A61K 31/505

(21) Application number : **90304979.9**

(22) Date of filing : **09.05.90**

(54) **Quinazoline derivatives.**

(30) Priority : **10.05.89 GB 8910722**
**16.08.89 GB 8918703**
**14.12.89 GB 8928251**

(43) Date of publication of application :
**27.12.90 Bulletin 90/52**

(45) Publication of the grant of the patent :
**22.09.93 Bulletin 93/38**

(84) Designated Contracting States :
**AT BE CH DE DK ES FR GB GR IT LI LU NL SE**

(56) References cited :
**EP-A- 0 028 473**
**EP-A- 0 322 133**
**US-A- 3 635 979**
**US-A- 4 098 788**
**US-A- 4 341 893**
**Journal of Medicinal Chemistry, 1978, vol. 21, p. 331**

(73) Proprietor : **SMITHKLINE BEECHAM INTERCREDIT B.V.**
**Jaagpad 1 P.O. Box 3120**
**NL-2280 GC Rijswijk (NL)**

(72) Inventor : **Brown, Thomas Henry**
**Smith Kline & French Research Ltd., The Frythe**
**Welwyn, Hertfordshire AL6 9AR (GB)**
Inventor : **Ife, Robert John**
**Smith Kline & French Research Ltd., The Frythe**
**Welwyn, Hertfordshire AL6 9AR (GB)**
Inventor : **Leach, Colin Andrew**
**Smith Kline & French Research Ltd., The Frythe**
**Welwyn, Hertfordshire AL6 9AR (GB)**
Inventor : **Keeling, David John**
**Smith Kline & French Research Ltd., The Frythe**
**Welwyn, Hertfordshire AL6 9AR (GB)**

(74) Representative : **Giddings, Peter John, Dr. et al**
**SmithKline Beecham, Corporate Patents, Mundells**
**Welwyn Garden City, Hertfordshire AL7 1EY (GB)**

EP 0 404 322 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to substituted quinazoline derivatives, processes for their preparation, intermediates useful in their preparation, pharmaceutical compositions containing them and their use in therapy.

Substituted quinazoline derivatives having utility as inhibitors of gastric acid secretion have been disclosed in EP-A 322 133. US 3,635,979 and US 4,098,788 both disclose a series of substituted quinazolines having utility in the treatment of hypertension. Further substituted quinazolines are disclosed in J. Med. Chem. 1978, **21**, 331 as antimalarial agents.

Accordingly the present invention provides, in a first aspect compounds of structure (I)

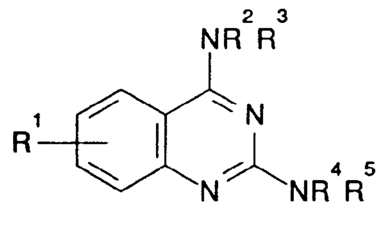

(I)

in which

$R^1$ is hydroxy, hydroxy$C_{1-4}$alkyl, hydroxy$C_{1-4}$alkoxy, carboxy$C_{1-4}$alkoxy,$C_{1-4}$alkoxycarbonyl$C_{1-4}$alkoxy, $C_{1-4}$alkoxy$C_{1-4}$alkoxy, $O(CH_2)_m$Het, $C_{1-4}$alkyl$NR^6R^7$ or $O(CH_2)_mNR^6R^7$ in which m is 2 to 4 and $R^6$ and $R^7$ are the same or different and are each hydrogen, $C_{1-4}$alkyl, Het, or $-(CH_2)_n$Ar where n is 0 to 4, Ar is a phenyl ring optionally substituted by 1 to 3 substituents selected from $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl, and Het is a 5- or 6-membered heterocyclic ring containing one or more heteroatoms, or $R^6$ and $R^7$ together with the nitrogen atom to which they are attached form a saturated or unsaturated ring optionally containing one or more further heteroatoms;

$R^2$ and $R^3$ are the same or different and are each hydrogen, $C_{1-4}$alkyl, $-(CH_2)_n$Ar in which n is 0 to 4 and Ar is a phenyl ring optionally substituted by 1 to 3 substituents selected from $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl, or $R^2$ and $R^3$ together with the nitrogen atom to which they are attached form a saturated or unsaturated ring optionally containing one or more further heteroatoms; and

$R^4$ and $R^5$ are the same or different and are each hydrogen, $C_{1-4}$alkyl, $(CH_2)_nAr^1$ in which n is 0 to 4 and $Ar^1$ is a phenyl ring optionally substituted by 1 to 3 substituents selected from $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl, or $R^4$ and $R^5$ together with the nitrogen atom to which they are attached form a saturated or unsaturated ring optionally containing one or more further heteroatoms;

provided that when $R^2$, $R^3$, $R^4$ and $R^5$ are all hydrogen $R^1$ is not $CH_2NR^6R^7$, and pharmaceutically acceptable salts thereof.

Suitably, $R^1$ is hydroxy, hydroxy$C_{1-4}$alkyl, carboxy-$C_{1-4}$alkoxy, hydroxy$C_{1-4}$alkoxy, $C_{1-4}$alkoxy$C_{1-4}$alkoxy, $O(CH_2)_m$Het, $C_{1-4}$alkyl$NR^6R^7$ or $-O(CH_2)_mNR^6R^7$; more suitably $R^1$ is hydroxy. Preferably $R^1$ is hydroxy, hydroxy$C_{1-4}$alkoxy or $O(CH_2)_mNR^6R^7$.

Suitably the group $R^1$ is in the 6, 7 or 8 position of the ring. Preferably the group $R^1$ is in the 6 or 8 position of the ring.

Suitably $R^2$ and $R^3$ are the same or different and are each hydrogen or $(CH_2)_n$Ar in which n is 0 to 4 and Ar is an optionally substituted phenyl ring or $R^2$ and $R^3$ together with the nitrogen atom to which they are attached form a saturated or unsaturated ring optionally containing one or more further heteroatoms. More suitably, one of $R^2$ and $R^3$ is hydrogen or $C_{1-4}$alkyl and the other is hydrogen, $C_{1-4}$alkyl or $(CH_2)_n$Ar. Most suitably one of $R^2$ and $R^3$ is hydrogen or $C_{1-4}$alkyl and the other is $(CH_2)_n$Ar. Preferably one of $R^1$ and $R^2$ is $C_{1-4}$alkyl and the other is $(CH_2)_n$Ar; most preferably one of $R^2$ and $R^3$ is $C_{1-4}$alkyl, in particular methyl and the other is $(CH_2)_n$Ar in which n is O and Ar is phenyl.

Suitably, Ar is unsubstituted or substituted by 1 to 3 substituents selected from $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl. More suitably,

Ar is unsubstituted or substituted by two substituents selected from $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl. More preferably, Ar is unsubstituted or substituted by two substituents selected from $C_{1-4}$alkyl and $C_{1-4}$alkoxy. Most preferably, Ar is unsubstituted or substituted by a single substituent selected from the above-noted groups, in particular hydroxy, halogen, $C_{1-4}$alkyl or $C_{1-4}$alkoxy.

Suitably, $R^4$ and $R^5$ are the same or different and are each hydrogen, $C_{1-4}$alkyl, $(CH_2)_nAr^1$ in which n is 0 to 4 and $Ar^1$ is an optionally substituted phenyl ring, or $R^4$ and $R^5$ together with the nitrogen atom to which they are attached form a saturated or unsaturated ring optionally containing one or more further heteroatoms. More suitably one of $R^4$ and $R^5$ is hydrogen or $C_{1-4}$alkyl and the other is hydrogen, $C_{1-4}$alkyl or $(CH_2)_nAr^1$, or $R^4$ and $R^5$ together with the nitrogen atom to which they are attached form a saturated or unsaturated ring optionally containing one or more further heteroatoms. Most suitably, one of $R^4$ and $R^5$ is hydrogen or $C_{1-4}$alkyl and the other is hydrogen, $C_{1-4}$alkyl or $(CH_2)_nAr^1$. Preferably one of $R^4$ and $R^5$ is hydrogen or $C_{1-4}$alkyl and the other is $(CH_2)_nAr^1$; more preferably one of $R^4$ and $R^5$ is hydrogen and the other is $(CH_2)_nAr^1$; most preferably, one of $R^4$ and $R^5$ is hydrogen and the other is $(CH_2)_nAr^1$ in which n is O and $Ar^1$ is a substituted phenyl ring.

Suitably, the group $Ar^1$ is unsubstituted or optionally substituted by 1 to 3 substituents as hereinabove described for Ar in $R^2$ or $R^3$. Preferably $Ar^1$ is unsubstituted or substituted by one or two substituents, for example $C_{1-4}$alkyl, in particular methyl, or a halogen atom, in particular fluorine; or $C_{1-4}$alkyl and halogen in particular methyl and fluorine. Particularly preferably $Ar^1$ is substituted by methyl in the 2-position of the ring and fluorine in the 4-position of the ring.

Suitably $R^6$ and $R^7$ are the same or different and are each hydrogen, $C_{1-4}$alkyl, Het or $(CH_2)_nAr$ in which suitable and preferred groups Ar are as hereinbefore defined for $R^2$ and $R^3$, or together with the nitrogen atom to which they are attached form a saturated or unsaturated ring optionally containing one or more further heteroatoms. Suitable rings include for example, morpholino rings. More suitably, $R^6$ and $R^7$ are the same and are each $C_{1-4}$alkyl.

Suitably n is 2 to 4, preferably n is 3.

$C_{1-4}$alkyl groups (either alone or as part of another group) can be straight or branched.

Carboxy$C_{1-4}$alkoxy groups are groups of structure $O(CH_2)_pCO_2R^8$ in which p is 1 to 4 and $R^8$ is hydrogen or $C_{1-4}$alkyl.

Het groups (either alone or as part of another group) include for example 5- or 6-membered heterocyclic rings containing one or more heteroatoms, for example imidazoyl and pyridyl groups.

It will be appreciated that compounds of structure (I) in which one or more of $R^1$ to $R^7$ is a $C_{3-4}$alkyl group (either alone or as part of another group) may contain an assymetric centre due to the presence of the $C_{3-4}$alkyl group. Such compounds will exist as two (or more) optical isomers (enantiomers). Both the pure enantiomers, racemic mixtures (50% of each enantiomer) and unequal mixtures of the two are included within the scope of the present invention. Further, all diastereomeric forms possible (pure enantiomers and mixtures thereof) are within the scope of the invention.

The compounds of the present invention can be prepared by processes analogous to those known in the art. The present invention therefore provides in a further aspect a process for the preparation of a compound of structure (I) or a pharmaceutically acceptable salt thereof which comprises

(a) reaction of a compound of structure (II)

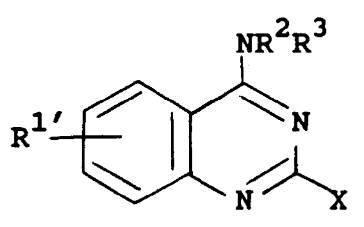

(II)

in which $R^2$ and $R^3$ are as described for structure (I), $R^{1'}$ is an optionally protected group $R^1$ and X is a group displaceable by an amine, with an amine of structure $R^4R^5NH$ in which $R^4$ and $R^5$ are as described for structure (I);

(b) reaction of a compound of structure (III)

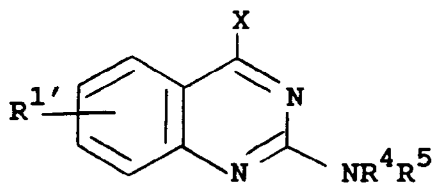

(III)

in which $R^{1'}$ is as described for structure (II), $R^4$ and $R^5$ are as described for structure (I) and X is a group displaceable by an amine, with an amine of structure $R^2R^3NH$ in which $R^2$ and $R^3$ are as described for structure (I);

(c) for compounds of structure (I) in which $NR^2R^3$ and $NR^4R^5$ are the same, reaction of a compound of structure (IV)

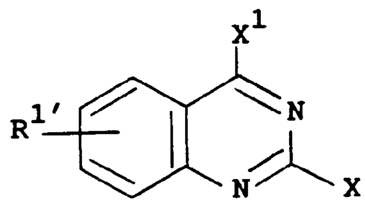

(IV)

in which $R^{1'}$ is as described for structure (II) and X and $X^1$ are both groups displaceable by an amine, with a compound of structure $R^2R^3NH$ or $R^4R^5NH$; or

(d) for compounds in which $R^1$ is other than hydroxy, $C_{1-4}alkylNR^6R^7$ or hydroxy$C_{1-4}$alkyl, alkylation of a compound of structure (V)

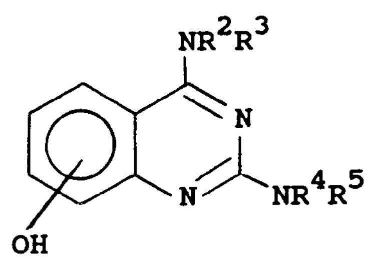

(V)

in which $R^2$ to $R^5$ are as described for structure (I); and optionally thereafter,

- ° removing any protecting groups;
- ° converting a group $R^{1'}$ into a group $R^1$;
- ° forming a pharmaceutically acceptable salt.

Suitable groups displaceable by an amine, X and $X^1$, will be apparent to those skilled in the art and include, for example, halogen, in particular chlorine, $SC_{1-4}$alkyl, such as methylthio, hydroxy and phenoxy.

Reaction of a compound of structure (II) with an amine $R^4R^5NH$ is suitably carried out in an inert solvent at elevated temperature. Preferably the reaction is carried out in the absence of a solvent in a sealed receptacle at elevated temperature. Most preferably in an inert solvent for example dioxan, at reflux temperature.

Reaction of a compound of structure (III) with an amine $R^2R^3NH$ and reaction of a compound of structure (IV) with a suitable amine is suitably carried out in the presence or absence of an inert solvent at elevated temperature. Suitable solvents include, for example $C_{1-4}$alkanols such as isopropanol or butanol, preferably isopropanol, dioxan or tetrahydrofuran.

In particular, leaving groups X and $X^1$ are halogen, preferably chlorine, and can be displaced by appropriate amines $R^2R^3NH$ and $R^4R^5NH$ under the general conditions described above and in the specific examples. Other conditions and reagents depending on the nature of the leaving groups will be apparent to those skilled in the art; for example compounds of structure (I) in which $R^2$ and $R^3$ are both hydrogen, can be prepared from the corresponding compounds of structure (III) in which X is hydroxy by reaction with phenylphosphordiamidate using the method described in J. Het. Chem (1972), 9, 1235.

The alkylation of compounds of structure (V) is carried out, for example, by reaction with a strong base,

such as sodium, in a suitable solvent, such as a $C_{1-4}$alkanol, in particular ethanol, followed by treatment with a suitable alkylating agent of structure $R^{10}X^3$ in which $R^{10}$ is hydroxy$C_{1-4}$alkyl or $(CH_2)_mNR^6R^7$ in which n, $R^6$ and $R^7$ are as described for structure (I), and $X^3$ is a leaving group, for example halogen, in particular chlorine or bromine. The alkylation reaction preferably is carried out in an inert solvent such as toluene, at a temperature of between ambient and reflux temperature of the solvent used, preferably at reflux temperature.

Pharmaceutically acceptable acid addition salts of the compounds of structure (I) can be prepared by standard procedures by, for example, reaction with suitable organic and inorganic acids the nature of which will be apparent to persons skilled in the art. For example, pharmaceutically acceptable salts can be formed by reaction with hydrochloric, sulphuric, or phosphoric acids; aliphatic, aromatic or heterocyclic sulphonic acids or carboxylic acids such as, for example, citric, maleic or fumaric acids, or methyl sulphonic acid.

The intermediate compounds of structure (II) and (III) can be prepared by procedures analogous to those known in the art. The amines of structure $R^2R^3NH$ and $R^4R^5NH$ are available commercially or can be prepared by standard techniques well known to those skilled in the art of organic chemistry.

For example compounds of structure (II) in which $R^{1'}$ is $OCH_3$, and X is chlorine can be prepared by the route outlined in Scheme I. It will be apparent to those skilled in the art that the reactions of Scheme 1 can also be carried out on compounds (A) in which $R^{1'}$ is other than $OCH_3$ to produce further compounds of structure (II).

## Scheme I

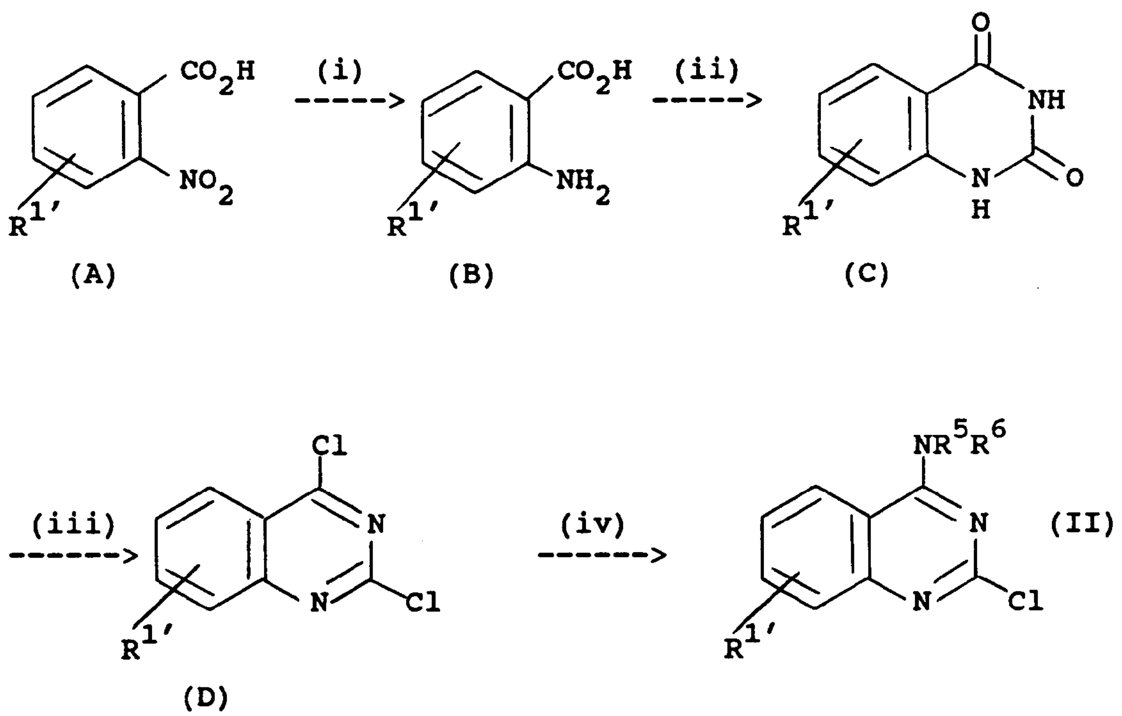


(A) (B) (C) (D) (II)

(i) $H_2$, Pd/C 10%, EtOH
(ii) KNCO, AcOH, NaOH
(iii) $POCl_3$, $PhNMe_2$, Δ
(iv) $R^5R^6NH$, NaOAc, $THF/H_20$, Δ.

It is to be noted that the reactions of Scheme I proceed via compounds of structure (IV), that is D in Scheme I.

Compounds of structure (III) in which $R^4$ and $R^5$ are both hydrogen, $C_{1-4}$alkyl or $(CH_2)_nAr^1$ or one is $C_{1-4}$alkyl and the other is $(CH_2)_nAr^1$ and X is chlorine can be prepared by the procedures outlined in Scheme II.

## Scheme II

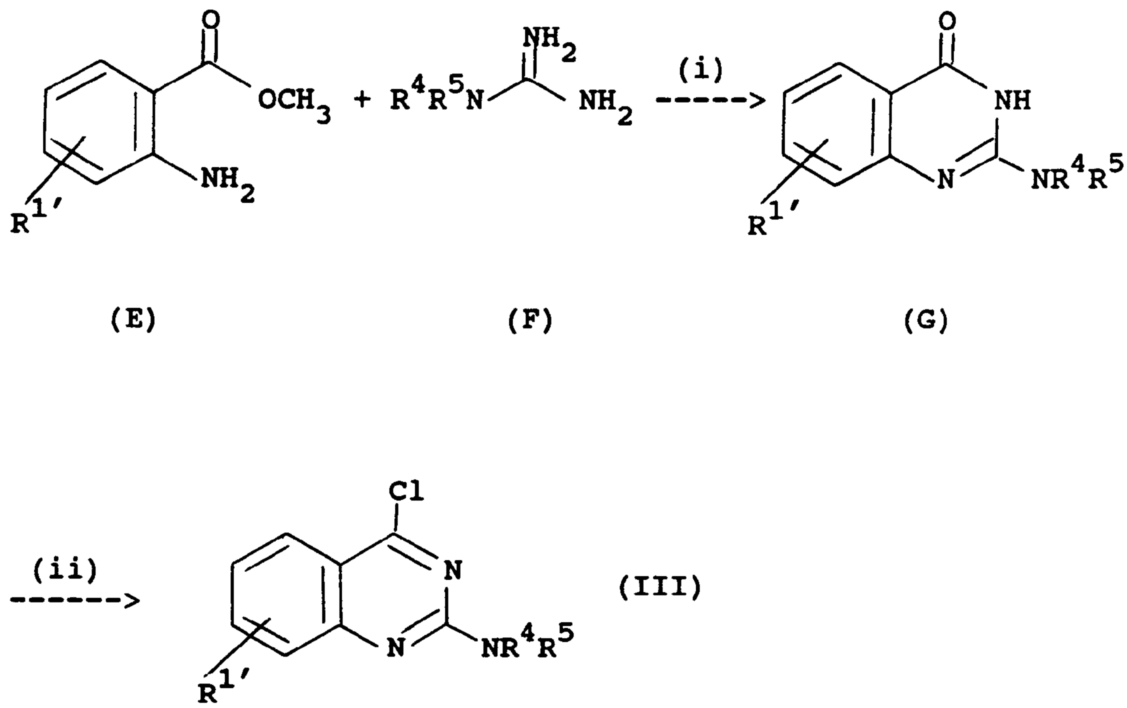


(E) (F) (G)

(III)

(i) $NaOCH_3$, nBuOH
(ii) $POCl_3$

Alternatively, compounds of structure (III) can be prepared from compounds of structure (D) - see Scheme I - by the method used in J. Med. Chem., 1981, 24, 127-140. This alternative method is outlined in Scheme III.

## Scheme III

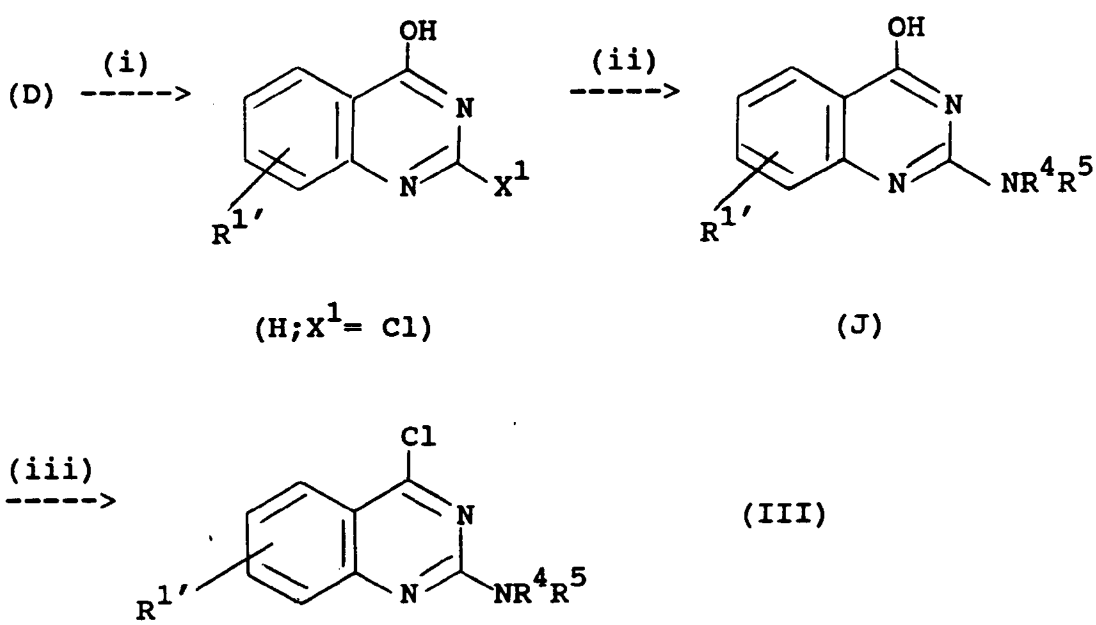


(D)

(H; $X^1$= Cl) (J)

(III)

(i) $NaOH/H_2O$
(ii) $R^4R^5NH$, solvent, Δ
(iii) $POCl_3$, solvent, Δ

Compounds of structure (V) can be prepared by the reactions outlined in the preceeding process options (a) to (c), for example those options can be used to prepare intermediate compounds in which $R^{1'}$ is a protected hydroxy group, for example, a methoxy group, which group can then be deprotected to form a hydroxy group. Conditions for such deprotections will be apparent to those skilled in the art and include, in particular treatment with boron tribromide in a suitable solvent such as dichloromethane at low temperature i.e. from about 0° to about 5°C.

The starting materials used to prepare compounds of structures (II) and (III) are available commercially or can be prepared by standard techniques. In addition it will be appreciated that further variations of the above-noted schemes can be utilized, for example, in Scheme III, compounds of structure (H) in which $X^1$ is Cl can be replaced by compounds of structure (H) in which $X^1$ is, for example methylthio and then converted to compounds of structure (III) as shown. Such compounds in which $X^1$ is methylthio are prepared by standard techniques for example by treatment of the analogous thione precursor with methyl iodide in ethanol in the presence of sodium hydroxide.

It is to be noted, and will be apparent to those skilled in the art that in the foregoing reactions, where necessary groups $R^1$ and groups on aromatic rings Ar and $Ar^1$ (e.g. hydroxy or amino groups) will be in "protected" form. For example, amino groups can be "protected" in the form of nitro groups and converted into amino groups as appropriate, and hydroxy groups can be protected using standard groups for example as described in "Greene, T.W., Protective Groups in Organic Chemistry" which also provides examples of further appropriate protective groups for other moities.

The compounds of structure (I) and their pharmaceutically acceptable salts exert an anti-secretory effect by inhibition of the gastrointestinal $H^+K^+$ATPase enzyme, and in addition have been found to be of use as inhibitors of bone resorption.

In a further aspect therefore the present invention provides compounds of structure (I) and pharmaceutically acceptable salts thereof for use in therapy.

The compounds of structure (I) and their pharmaceutically acceptable salts inhibit exogenously and endogenously stimulated gastric acid secretion and are useful in the treatment of gastrointestinal diseases in mammals, in particular humans. Such diseases include, for example, gastric and duodenal ulcers and Zollinger-Ellison Syndrome. Further, the compounds of structure (I) can be used in the treatment of other disorders where an anti-secretory effect is desirable for example in patients with gastritis, NSAID induced gastritis, gastric ulcers, acute upper intestinal bleeding, in patients with a history of chronic and excessive alcohol consumption, and in patients with gastro oesophageal reflux disease (GERD).

In addition the compounds of structure (I) have been found to be of use in medicine as inhibitors of bone resorption. In normal subjects there is a balance between bone resorption and bone formation, however in subjects with bone affected diseases such as osteoporosis, Paget's disease and hyperparathyroidism and related disorders this balance is disturbed. As a consequence the subject suffers a loss of bone tissue, decreased bone mass and bone fragility which can result in fracturing of bones. Bone resorption (or bone loss) is associated with the activity of osteoclast cells, and it is thought that agents which inhibit the activity of such cells (and so inhibit bone resorption) will have a beneficial effect on the reduction of bone loss and be of benefit in the treatment of the above-noted disease states. The present compounds have been found to be inhibitors of osteoclast activity and bone resorption and are expected to be of use in medicine in the treatment of diseases in which bone loss is a factor, in particular osteoporosis, Paget's disease and hyperparathyroidism.

In therapeutic use, the compounds of the present invention are usually administered in a standard pharmaceutical composition.

The present invention therefore provides in a further aspect pharmaceutical compositions comprising a compound of structure (I) or a pharmaceutically acceptable salt thereof and a pharmaceutically acceptable carrier.

The compounds of structure (I) and their pharmaceutically acceptable salts which are active when given orally can be formulated as liquids, for example syrups, suspensions or emulsions, tablets, capsules and lozenges.

A liquid formulation will generally consist of a suspension or solution of the compound or pharmaceutically acceptable salt in a suitable liquid carrier(s) for example, ethanol, glycerine, non-aqueous solvent, for example polyethylene glycol, oils, or water with a suspending agent, preservative, flavouring or colouring agent.

A composition in the form of a tablet can be prepared using any suitable pharmaceutical carrier(s) routinely used for preparing solid formulations. Examples of such carriers include magnesium stearate, starch, lactose, sucrose and cellulose.

A composition in the form of a capsule can be prepared using routine encapsulation procedures. For example, pellets containing the active ingredient can be prepared using standard carriers and then filled into a hard gelatin capsule; alternatively, a dispersion or suspension can be prepared using any suitable pharmaceutical carrier(s), for example aqueous gums, celluloses, silicates or oils and the dispersion or suspension then filled into a soft gelatin capsule.

Typical parenteral compositions consist of a solution or suspension of the compound or pharmaceutically acceptable salt in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

A typical suppository formulation comprises a compound of formula (I) or a pharmaceutically acceptable salt thereof which is active when administered in this way, with a binding and/or lubricating agent such as polymeric glycols, gelatins or cocoa butter or other low melting vegetable or synthetic waxes or fats.

Preferably the composition is in unit dose form such as a tablet or capsule.

Each dosage unit for oral administration contains preferably from 1 to 250 mg (and for parenteral administration contains preferably from 0.1 to 25 mg) of a compound of the formula (I) or a pharmaceutically acceptable salt thereof calculated as the free base.

The pharmaceutically acceptable compounds of the invention will normally be administered in a daily dosage regimen (for an adult patient) of, for example, an oral dose of between 1 mg and 500 mg, preferably between 1 mg and 250 mg, or an intravenous, subcutaneous, or intramuscular dose of between 0.1 mg and 100 mg, preferably between 0.1 mg and 25 mg, of the compound of the formula (I) or a pharmaceutically acceptable salt thereof calculated as the free base, the compound being administered 1 to 4 times per day. Suitably the compounds will be administered for a period of continuous therapy, for example for a week or more.

In addition, the compounds of the present invention can be co-administered with further active ingredients in particular when used to treat conditions caused or exacerated by gastric acidity. Such ingredients include antacids (for example magnesium carbonate or hydroxide and aluminium hydroxide), non-steroidal anti-flammatory drugs (for example indomethacin, aspirin or naproxen), steroids, or nitrite scavengers (for example ascorbic acid or aminosulphonic acid), or other drugs used for treating gastric ulcers (for example pirenzipine, prostanoids for example 16,16 dimethyl $PGE_2$, or histamine $H_2$-antagonists (for example, cimetidine).

The following examples illustrate the invention. Temperatures are recorded in degrees centigrade.

Example 1

4-(N-Methylphenylamino)-2-(2-methylphenylamino)-8-hydroxyquinazoline.

4-(N-Methylphenylamino)-2-(2-methylphenylamino)-8-methoxyquinazoline (prepared as described in co-pending EP-A-322 133) (1.81 g, 0.0049 mol) was dissolved in dichloromethane (50 ml) and cooled to 0-5° under nitrogen. Boron tribromide (6.25 g, 0.025 mol) was added dropwise via a syringe through a rubber septum over a period of 10 minutes. Reaction was stirred 3 hours at 0-5° and then allowed to rise to room temperature overnight. The reaction mixture was poured onto ice and the pH adjusted to ~12 with sodium hydroxide and then back to neutral with dilute HCl. The organic layer was separated, dried and evaporated to dryness. The solid residue was crystallised from methanol/water to give the title compound (0.66 g), m.p. 174-175°.

| | $C_{22}H_{20}N_4O$. 0.4 $H_2O$ |
|---|---|
| Found | C 72.69, H 5.71, N 15.38 |
| Requires | C 72.67, H 5.77, N 15.41 |

Example 2

Bis-2,4-(N-methylphenylamino)-8-hydroxyquinazoline hydrochloride

Bis-2,4-(N-metlylphenylamino)-8-methoxyquinazoline (prepared as described in co-pending EP-A-322 133) (4.5 g, 0.013 mol) was suspended in dichloromethane (100 ml) and cooled to 0-5° in an atmosphere of nitrogen. Boron tribromide (16.5 g, 0.066 mol) was added dropwise from a syringe through a rubber septum. The reaction was stirred for three hours at 0-5°, allowed to rise to room temperature overnight and poured onto ice. The aqueous phase was basified with sodium hydroxide and then neutralised with dilute HCl. The organic layer was separated, washed with water, dried and evaporated to dryness. The residue was chromatrographed

on silica gel in ether. The product in ether was acidified with ethanolic hydrogen chloride (ethanol saturated with HCl gas) and the solid produced collected and recrystallised from isopropanol/ether to give the title compound (0.52 g) as its hydrochloride salt, m.p. 208-210°.

| | $C_{22}H_{20}N_4O$. HCl. 0.5 $H_2O$ |
|---|---|
| Found | C 65.97, H 5.37, N 13.62 |
| Requires | C 65.75, H 5.52, N 13.94 |

Example 3

4-(N-Methylphenylamino)-2-[(2-methyl-4-fluorophenyl)amino]-8-hydroxyquinazoline hydrobromide

4-(N-Methylphenylamino)-2-[(2-methyl-4-fluorophenyl)amino]-8-methoxyquinazoline (prepared as described in co-pending EP-A-322 133) (6.75 g, 0.016 mol) was suspended in dichloromethane (150 ml) and the mixture cooled to 0-5° under nitrogen. Boron tribromide (20 g, 0.08 mol) was added dropwise over 10 minutes with a syringe, keeping the temperature at 0-5°. The mixture was stirred at room temperature overnight. Reaction was incomplete (t.l.c) so the mixture was heated at reflux temperature for eight hours, cooled and poured onto ice. The mixture was basified (NaOH) and then neutralised (dilute HCl), when an insoluble solid precipitated which was collected, washed with water, dried and crystallised from methanol to yield the title compound (2.8 g) as its hydrobromide salt, m.p. >300°.

| | $C_{22}H_{19}FN_4O$. HBr. |
|---|---|
| Found | C 58.02, H 4.39, N 12.12 |
| Requires | C 58.03, H 4.43, N 12.31 |

Example 4

8-(3-Dimethylaminopropoxyl-2-[(2-methylphenyl)amino]-4-(N-methylphenylamino)quinazoline

Sodium metal (103 mg, 4.49 mM) was dissolved in absolute ethanol (20 ml) and the solution added to a suspension of 2-[(2-methylphenyl)amino]-4-(N-methylphenylamino)-8-hydroxyquinazoline (1.6 g, 4.49 mM) in ethanol (100 ml). The mixture was heated until a clear solution was produced and than the ethanol was evaporated off. Toluene (25 ml) was added and again the mixture was evaporated to dryness. The residue was dissolved in toluene (50 ml) and to this solution was added a solution of N,N-dimethylaminopropylchloride (546 mg, 4.49 mM) in toluene (75 ml). The resulting mixture was heated at reflux temperature overnight, cooled, washed with water (x 2), dried and evaporated to dryness, to give a browny-yellow oil. This oil was crystallised from ether/pentane to give the title compound (0.9 g) as buff-coloured flakes, m.p. 77-79°.

| | $C_{27}H_{31}N_5O$ |
|---|---|
| Found | C 73.27, H 7.17, N 15.64 |
| Requires | C 73.44, H 7.08, N 15.86 |

Example 5

4-(N-Methylphenylamino)-2-[(2-methylphenyl)amino]-6-hydroxyquinazoline hydrobromide

4-(N-Methylphenylamino)-2-[(2-methylphenyl)amino]-6-methoxyquinazoline (prepared as described in co-pending EP-A-322 133) (3.0 g, 0.0074 mol) and boron tribromide (9.2 g, 0.037 mol) were reacted together under the conditions described in Example 3 to give the title compound (1.39 g), crystallised from methanol/diethyl ether as its hydrobromide salt, m.p. 172-174°.

$C_{22}H_{20}N_4O$. HBr. $0.8H_2O$

| | |
|---|---|
| Found | C 58.61, H 5.04, N 12.39 |
| Requires | C 58.49, H 5.04, N 12.40 |

Example 6

4-(N-methylphenylamino)-2-[(2-methyl-4-fluorophenyl)amino]-6-hydroxyquinazoline hydrochloride

4-(N-methylphenylamino)-2-[(2-methyl-4-fluorophenyl)amino]-6-methoxyquinazoline (prepared as described in co-pending EP-A-322 133) (3.0 g, 0.007 mol) and boron tribromide (8.75 g, 0.035 mol) were reacted together and the product worked-up as described in Example 2. This gave the title compound as its hydrochloride salt (0.26 g) crystallised from isopropanol/ether, m.p. 258-260°.

$C_{22}H_{19}FN_4O$. HCl (+2.5% I.P.A.)

| | |
|---|---|
| Found | C 63.03, H 5.05, N 12.80 |
| Requires | C 62.92, H 5.09, N 12.81 |

Example 7

4-(N-methylphenylamino)-2-[(2-methyl-4-fluorophenyl)amino]-6-(3-dimethylaminopropoxy)quinazoline

To a suspension of 4-(N-methylphenylamino)-2-[(2-methyl-4-fluorophenylamino]-6-hydroxyquinazoline hydrochloride (2.05 g, 0.005 mol) in ethanol (20 ml) was added a solution of sodium (0.23 g, 0.01 mol) in ethanol (15 ml). The resulting solution was evaporated to dryness, and the residue dissolved in dry toluene (25 ml) and brought to reflux temperature. To this solution was added a freshly prepared solution of N,N-dimethylaminopropylchloride in toluene (25 ml) (from 1.58 g, 0.01 mol of the hydrochloride) over 15 minutes under nitrogen. The reaction was heated at reflux temperature overnight, cooled, washed with aqueous $Na_2CO_3$ and water, dried and evaporated to dryness to give an oily residue. This was purified by column chromatography on silica gel using first ethyl acetate and then ethyl acetate/methanol, 5:1 as eluent. Fractions containing required product (pure by t.l.c) were combined, evaporated to dryness to give an oil which solidified under petroleum ether to give the title compound (0.6 g), m.p. 103-104°.

$C_{27}H_{30}FN_5O$ (+ 1% $H_2O$)

| | |
|---|---|
| Found | C 69.85, H 6.37, N 14.93 |
| Requires | C 69.85, H 6.62, N 15.08 |

Example 8

4-(N-methylphenylamino)-2-[(2-methyl-4-fluorophenyl)amino]-6-(3-morpholinopropoxy)quinazoline

Substituting 3-morpholinopropylchloride (from 2.0 g, 0.01 mol of hydrochloride) for N,N-dimethylaminopropyl chloride and using corresponding molar proportions of the other reagents in Example 7 gave, after removal of the toluene, an oily solid. This was crystallised from diethyl ether/pentane to give the title compound (1.34 g), m.p. 119-120°.

$C_{29}H_{32}FN_5O_2$

| | |
|---|---|
| Found | C 69.71, H 6.40, N 13.66 |
| Requires | C 69.44, H 6.43, N 13.96 |

Example 9

4-(N-methylphenylamino)-2-[(2-methyl-4-fluorophenyl)amino]-6-(2-dimethylaminoethoxy)quinazoline

Substituting N,N-dimethylaminoethylchloride (from 1.44 g, 0.01 mole of hydrochloride) from N,N-dimethylaminopropylchloride and using corresponding molar proportions of the other reagents in Example 7 gave, after cooling, the reaction mixture in toluene. This was worked with water, followed by 2N HCl. The HCl washings were basified with 2N.NaOH and extracted with dichloromethane. The dichloromethane was washed with water, dried and evaporated to dryness. The residue was purified by column chromatography on silica gel using ethyl acetate/methanol, 10:1 as eluent. Combination of the appropriate fractions (t.l.c) and evaporation gave an oily residue. This was crystallised from diethyl ether/pentane to yield the title compound (0.73 g), m.p. 112-114°.

| | $C_{26}H_{28}FN_5O$ |
|---|---|
| Found | C 70.14, H 6.07, N 15.47 |
| Requires | C 70.09, H 6.33, N 15.72 |

Example 10

8-(Hydroxyethoxy)-4-(N-methylphenylamino)-2-[(2-methylphenyl)amino]quinazoline

(i) Sodium hydride (0.325 g of 50% dispersion in oil 6.75 mmol) was added to dry DMF (30 ml) and to the stirred solution under dry nitrogen was added a solution of 8-hydroxy-2-[(2-methylphenyl)amino]-4-(N-methylphenylamino)quinazoline (2.4 g, 6.75 mmol) in dry DMF (30 ml). After stirring at room temperature for one hour (solution goes from greeny-yellow to red-brown) a solution of ethyl bromoacetate (1.125 g, 6.75 mmol) in dry DMF (30 ml) was added dropwise over 0.5 hour. The resulting mixture was stirred at room temperature for four hours, water added followed by aq. $Na_2CO_3$. The mixture was extracted with ethyl acetate (x 2) and the organic extracts combined, washed with water, dried and evaporated to give a green oil (2.9 g).

This material was chromatographed on silica gel using $CH_2Cl_2$ as initial eluent. Fractions were monitored by thin-layer chromatography and the major product was eluted with $CH_2Cl_2$/1% methanol. Relevant fractions were combined and evaporated to dryness to give 8-(ethoxycarbonylmethoxy)-4-(N-methylphenylamino)-2-[(2-methylphenyl)amino]quinazoline as a green glass (2.4 g).

(ii) Lithium aluminium hydride (0.27 g, 7.1 mmol) was added to dry distilled THF (50 ml) and the mixture stirred in an ice-bath under dry nitrogen. A solution of 8-(ethoxycarbonylmethoxy)-4-(N-methylphenylamino)-2-[(2-methylphenyl)amino]quinazoline (2.4 g, 5.43 mmol) in dry THF (30 ml) was added dropwise at such a rate as to keep the reaction temperature below 5°. The mixture was then allowed to freely reach room temperature and stirred for three hours. Water was carefully added followed by a little 40% NaOH. Two layers were produced and this mixture was extracted (x 2) with ethyl acetate. The combined organic extracts were worked with water (x 3) and brine, dried ($Na_2SO_4$) and evaporated to give an oil which crystallised on scratching to a yellow solid (1.9 g).

This material was crystallised twice from acetone/diethyl ether to give the title compound (0.66 g) as a yellow solid, m.p. 158-160°.

| | $C_{24}H_{24}N_4O_2$ |
|---|---|
| Found | C 71.87, H 6.22, N 13.60 |
| Requires | C 71.98, H 6.04, N 13.99 |

Example 11

2-(2-Methyl-4-fluorophenylamino)-4-(N-methylphenylamino)-8-(3-morpholinopropoxy)quinazoline

2-(2-Methyl-4-fluorophenylamino)-4-(N-methylphenylamino)-8-hydroxyquinazoline (1.6 g, 4.3 mmol) was suspended in ethanol (25 ml) and to the stirred suspension was added a solution of sodium (0.1 g, 4.3 mmol)

in ethanol (15 ml). Stirring was continued for 15 minutes and the mixture evaporated to dryness. The residue was dissolved in toluene (50 ml) and heated to reflux temperature. To this refluxing solution was added a freshly prepared solution of 3-morpholinopropyl chloride base (from 1.28 g, 6.4 mmol of hydrychloride salt) in toluene (20 ml). Refluxing was continued overnight and after cooling the mixture was washed with sodium carbonate solution and water, dried and evaporared. The residual oil was chromatographed on silica gel using $CH_2Cl_2$ as eluting solvent. After evaporation of the relevent fractions the residue was crystallised from diethyl ether/pentane to give the title compound (1.5 g, 71%) m.p. 133-134°.

| | $C_{29}H_{32}FN_5O_2$ |
|---|---|
| Found | C 69.23, H 6.54, N 13.82 |
| Requires | c 69.44, B 6.43, N 13.96 |

Example 12

2-(2-Methyl-4-fluorophenylamino)-4-(N-methylphenylamino)-8-(3-dimethylaminopropoxy)quinazoline

Substituting N,N-dimethylaminpropyl chloride hydrochloride (1.6 g, 8.6 mmol) for 3-morpholinopropyl chloride hydrochloride in Example 11 and using corresponding molar proportions of the other reagents gave an oily solid after chromatography. This was crystallised from diethylether/pentane to give the title compound (1.04 g, 53%), m.p. 110-112°.

| | $C_{27}H_{30}FN_5O$ |
|---|---|
| Found | C 70.51, H 6.55, N 15.01 |
| Requires | C 70.56, H 6.58, N 15.24 |

Example 13

8-(Ethoxycarbonylmethoxy)-4-(N-methylphenylamino)-2-[(2-methyl-4-fluorophenyl)amino]quinazoline)

To a suspension of sodium hydride (0.22 g, 0.0048 mol) in dimethylformamide (30 ml) was added a solution of 2-[(2-methyl-4-fluorophenyl)amino]-4-(N-methylphenylamino)-8-hydroxyquinazoline (1.8 g, 0.0048 mol) in dimethylformamide (10 ml). The reaction mixture was stirred for two hours at room temperature and then a solution of ethyl bromoacetate (0.9 g, 0.0048 mol) in DMF (10 ml) was added slowly. The mixture was stirred at room temperature for a further 4 hours and then water (50 ml) added followed by aqueous sodium bicarbonate. The basic mixture was then extracted with ethyl acetate, the organic extracts washed, dried and evaporated to dryness to give the title compound as a buff crystalline solid (1.4 g, m.p. 134-136°).

| | $C_{26}H_{25}FN_4O_3$. 0.24 $H_2O$. |
|---|---|
| Found | C 67.0, H 5.3, N 12.0 |
| Requires | C 67.2, H 5.5, N 12.1 |

Example 14

8-(Hydroxyethoxy)-4-]N-methylphenylamino)-2-[(2-methyl-4-fluorophenyl)amino]quinazoline

To dry, stirred tetrahydrofuran (40 ml) cooled in an ice-bath under an atmosphere of nitrogen was added lithium aluminium hydride (0.15 g, 0.004 mol) over a period of 10 minutes. The reaction mixture was refluxed for six hours, cooled and water (20 ml) carefully added followed by 40% NaOH. The mixture was extracted with ethyl acetate and the combined organic extracts washed, dried and evaporated to dryness. The residue was chromatographed on silica gel using ethyl acetate as mobile phase. Appropriate fractions (t.l.c.) were combined and evaporated and the residue crystallised from isopropyl alcohol/pentane to give the title compound (0.04 g) as the hemi-hydrate, m.p. 148-150°.

$C_{24}H_{23}FN_4O_2 . 0.5 H_2O.$

| | |
|---|---|
| Found | C 67.6, H 5.7, N 12.7 |
| Requires | C 67.4, H 5.7, N 13.1 |

Biological Data.

(A) $H^+K^+$ATPase Activity.

The effects of a single high concentration (100 μM) of a compound of structure (I) on K-stimulated ATPase activity in lyophilised gastric vesicles was determined. Preferred compounds of structure (I) were also tested over a range of concentrations to determine $IC_{50}$ values.

(i) Preparation of lyophilised gastric vesicles (H/K-ATPase).

Lyophilised gastric vesicles were prepared from pig fundic mucosa after the method of Keeling et. al. (Biochem. Pharmacol., 34, 2967, 1985).

(ii) $K^+$-stimulated ATPase activity.

$K^+$-stimulated ATPase activity was determined at 37° in the presence of the following : 10 mM Pipes/Tris buffer pH 7.0, 2 mM $MgSO_4$, 1 mM KCl, 2 mM $Na_2$ATP and 3-6 μg protein/ml lyophilised gastric vesicles. After incubation for 30 minutes, the inorganic phosphate hydrolysed from ATP was determined by the method of Yoda and Hokin (Biochem. Biophys. Res. Commun. 40, 880, 1970).

Compounds of structure (I) were dissolved in dimethylsulphoxide which up to the highest concentration used had no effect on $K^+$-stimulated ATPase activity.

The effect of the highest concentration of each compound of structure (I) on the recovery of a standard amount of inorganic phosphate was also determined.

(iii) Results.

The compounds of the examples had $IC_{50}$ values in the range of from 0.018 to 1.3 μM.

(B) Bone resorption inhibitory activity

Bone resorption was measured by the actions of isolated rat osteoclasts on cortical bone slices (Zaidi et al Quarterly Journal of Experimental Physiology 1988 73:471-485 . Newborn Wistar rats were killed by decapitation and their femora and tibiae removed. Osteoclasts were mechanically disaggregated by curetting the bones into medium followed by agitation with a pipette. Osteoclasts were separated from other cells by sedimentation onto bone slices (15 minutes, 37°) after which the slices were removed and gently washed. The bone slices were then incubated in the presence of test compounds (37°, 10% humidified CO2, 18 hours) and fixed with glutaraldehyde. The degree of bone resorption was then assessed by the area of erosions produced by the osteoclasts using scanning electron microscopy. Data were meaned from measurements of 6 bone slices in each of two independent experiments.

Test compounds were dissolved in ethanol which was also added to control incubations (1%) in the absence of compound.

| | Conc. (μM) | Bone resorption (%) |
|---|---|---|
| Control | | 100 ± 27 |
| Example 6 | 10 | 7 ± 4 |
| | 100 | 7 ± 0 |

Mean ± SEM, n=12

Example A

A tablet for oral administration is prepared by combining

| | mg/Tablet |
|---|---|
| Compound of structure (I) | 100 |
| lactose | 153 |
| Starch | 33 |
| crospovidone | 12 |
| microcrystalline cellulose | 30 |
| magnesium stearate | 2 |
| | 330 mg |

into a 9 mm tablet.

Example B

An injection for parenteral administration is prepared from the following

| | | %w:w |
|---|---|---|
| Compound of structure (I) | | 0,50% (w:v) |
| 1M citric acid | | 30% (v:v) |
| sodium hydroxide (qs) | | to pH 3.2 |
| water for injection EP | to | 100 ml |

The compound of structure (I) is dissolved in the citric acid and the pH slowly adjusted to pH 3.2 with the sodium hydroxide solution. The solution was then made up to 100 ml with water, sterilised by filtration and sealed into appropriately sized ampoules and vials.

## Claims

1. A compound of structure (I)

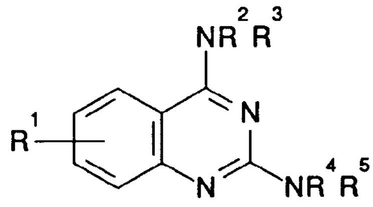

(I)

in which

$R^1$ is hydroxy, hydroxy$C_{1-4}$alkyl, hydroxy$C_{1-4}$alkoxy, carboxy$C_{1-4}$alkoxy, $C_{1-4}$alkoxycarbonyl$C_{1-4}$alkoxy, $C_{1-4}$alkoxy$C_{1-4}$alkoxy, $O(CH_2)_m$Het, $C_{1-4}$alkyl$NR^6R^7$ or $O(CH_2)_mNR^6R^7$ in which m is 2 to 4 and $R^6$ and $R^7$ are the same or different and are each hydrogen, $C_{1-4}$alkyl, Het, or $-(CH_2)_nAr$ where n is 0 to 4, Ar is a phenyl ring optionally substituted by 1 to 3 substituents selected from $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl, and Het is a 5- or 6-membered heterocyclic ring containing one or more heteroatoms, or $R^6$ and $R^7$ together with the nitrogen atom to which they are attached form a saturated or unsaturated ring optionally containing one or more further heteroatoms;

$R^2$ and $R^3$ are the same or different and are each hydrogen, $C_{1-4}$alkyl, $-(CH_2)_nAr$ in which n is 0 to 4 and Ar is a phenyl ring optionally substituted by 1 to 3 substituents selected from $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl, or $R^2$ and $R^3$ together with the nitrogen atom to which they are attached form a saturated or unsaturated ring optionally containing one or more further heteroatoms; and

$R^4$ and $R^5$ are the same or different and are each hydrogen, $C_{1-4}$alkyl, $(CH_2)_nAr^1$ in which n is O to 4 and $Ar^1$ is a phenyl ring optionally substituted by 1 to 3 substituents selected from $C_{1-4}$alkyl, $C_{1-4}$alkoxy, $C_{1-4}$alkylthio, halogen, cyano, amino, hydroxy, carbamoyl, carboxy, $C_{1-4}$alkanoyl or trifluoromethyl, or $R^4$ and $R^5$ together with the nitrogen atom to which they are attached form a saturated or unsaturated ring optionally containing one or more further heteroatoms;

provided that when $R^2$, $R^3$, $R^4$ and $R^5$ are all hydrogen $R^1$ is not $CH_2NR^6R^7$, and pharmaceutically acceptable salts thereof.

2. A compound according to claim 1 in which one of $R^2$ and $R^3$ is $(CH_2)_nAr$ in which n is O and Ar is phenyl, and the other is methyl, and one of $R^4$ and $R^5$ is hydrogen and the other is $(CH_2)_nAr^1$ in which n is O and $Ar^1$ is a substituted phenyl ring.

3. A compound according to claim 2 in which $Ar^1$ is substituted by a methyl group in the two position, or a methyl group in the two position and a fluorine group in the 4-position.

4. A compound according to claim 3 in which $R^1$ is hydroxy.

5. A compound according to claim 3 in which $R^1$ is hydroxy$C_{1-4}$alkoxy or $O(CH_2)_mNR^6R^7$ in which m, $R^6$ and $R^7$ are as described for structure I in claim 1.

6. A compound according to claim 1 which is
4-(N-methylphenylamino)-2-(2-methylphenylamino)-8-hydroxyquinazoline,
bis-2,4-(N-methylphenylamino)-8-hydroxyquinazoline hydrochloride,
4-(N-methylphenylamino)-2-[(2-methyl-4-fluorophenyl)amino]8-hydroxyquinazoline hydrobromide,
8-(3-dimethylaminopropoxy)-2-[(2-methylphenyl)amino]-4-(N-methylphenylamino)quinazoline,
4-(N-methylphenylamino)-2-[(2-methylphenyl)amino]-6-hydroxy-quinazoline hydrobromide,
4-(N-methylphenylamino)-2-[(2-methyl-4-fluorophenyl)amino]-6-hydroxyquinazoline hydrochloride,
4-(N-methylphenylamino)-2-[(2-methyl-4-fluorophenyl)amino]-6-(3-dimethylaminopropoxy)quinazoline,
4-(N-methylphenylamino)-2-[(2-methyl-4-fluorophenyl)amino]-6-(3-morpholinopropoxy)quinazoline,
4-(N-methylphenylamino)-2-[(2-methyl-4-fluorophenyl)-amino]-6-(2-dimethylaminoethoxy)quinazoline,
8-(hydroxyethoxy)-4-(N-methylphenylamino)-2-[(2-methylphenyl)amino]quinazoline,
2-(2-methyl-4-fluorophenylamino)-4-(N-methylphenyl-amino)-8-(3-morpholinopropoxy)quinazoline,
2-(2-methyl-4-fluorophenylamino)-4-(N-methylphenyl-amino)-8-(3-dimethylaminopropoxy)quinazoline,
or a pharmaceutically acceptable salt thereof.

7. A pharmaceutical composition comprising a compound according to any one of claims 1 to 6 and a pharmaceutical carrier.

8. A compound according to any one of claims 1 to 6 for use as a therapeutic agent.

9. A process for the preparation of a compound according to claim 1 which comprises :

(a) reaction of a compound of structure (II)

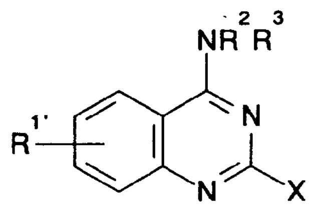

(II)

in which $R^2$ and $R^3$ are as described for structure (I), $R^{1'}$ is an optionally protected group $R^1$ and X is a group displaceable by an amine, with an amine of structure $R^4R^5NH$ in which $R^4$ and $R^5$ are as described for structure (I);

(b) reaction of a compound of structure (III)

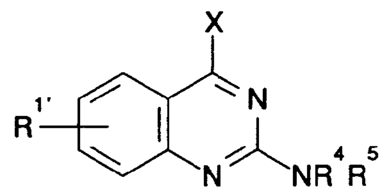

(III)

in which $R^{1'}$ is as described for structure (II), $R^4$ and $R^5$ are as described for structure (I) and X is a group displaceable by an amine, with an amine of structure $R^2R^3NH$ in which $R^2$ and $R^3$ are as described for structure (I); or

(c) for compounds of structure (I) in which $NR^2R^3$ and $NR^4R^5$ are the same, reaction of a compound of structure (IV)

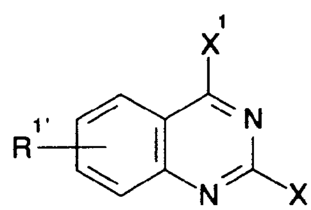

(IV)

in which $R^{1'}$ is as described for structure (II) and X and $X^1$ are both groups displaceable by an amine, with a compound of structure $R^2R^3NH$ or $R^4R^5NH$;

(d) for compounds in which $R^1$ is other than hydroxy, $C_{1-4}alkylNR^6R^7$ or hydroxy$C_{1-4}$alkyl, alkylation of a compound of structure (V)

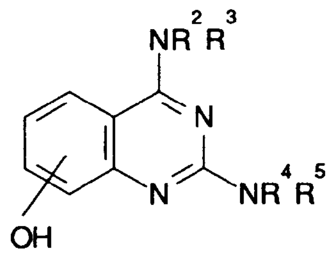

(V)

in which $R^2$ to $R^5$ are as described for structure (I); and optionally thereafter,

- ° removing any protecting groups;
- ° converting one group $R^{1'}$ into another group $R^1$;
- ° forming a pharmaceutically acceptable salt.

**10.** A compound of structure (II), (IV) or (V) as described in claim 9, provided that $R^{1'}$ is not alkoxy in compounds of formulae (II) and (IV).

**Patentansprüche**

**1.** Verbindung der Struktur (I)

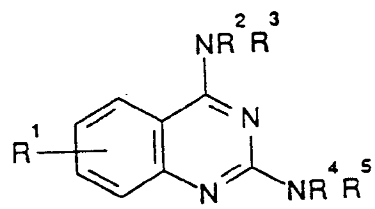

(I)

in der

$R^1$ eine Hydroxylgruppe, einen Hydroxy-$C_{1-4}$-alkyl-, Hydroxy-$C_{1-4}$-alkoxy-, Carboxy-$C_{1-4}$-alkoxy-, $C_{1-4}$-Alkoxycarbonyl-$C_{1-4}$alkoxy-, $C_{1-4}$-Alkoxy-$C_{1-4}$-alkoxyrest oder einen Rest der Formel $O(CH_2)_m$Het, $C_{1-4}$-Alkyl$NR^6R^7$ oder $O(CH_2)_mNR^6R^7$ bedeutet, wobei m 2 bis 4 ist und $R^6$ und $R^7$ gleich oder verschieden sind und jeweils ein Wasserstoffatom, einen $C_{1-4}$-Alkylrest, Het oder den Rest $-(CH_2)_nAr$ bedeuten, wobei n 0 bis 4 ist, Ar einen Phenylring bedeutet, gegebenenfalls substituiert mit 1 bis 3 Substituenten, ausgewählt aus $C_{1-4}$-Alkyl-, $C_{1-4}$-Alkoxy- oder $C_{1-4}$-Alkylthioresten, Halogenatomen, Cyano-, Amino-, Hydroxy-, Carbamoyl- oder Carboxylgruppen, $C_{1-4}$-Alkanoylresten oder Trifluormethylgruppen, und Het einen 5 bis 6-gliedrigen heterocyclischen Ring mit einem oder mehreren Heteroatomen bedeutet, oder $R^6$ und $R^7$ zusammen mit dem Stickstoffatom, an das sie gebunden sind, einen gegebenenfalls einen oder mehrere weitere Heteroatome enthaltenden gesättigten oder ungesättigten Ring bilden;

$R^2$ und $R^3$ gleich oder verschieden sind und jeweils ein Wasserstoffatom, einen $C_{1-4}$-Alkylrest oder einen Rest der Formel $-(CH_2)_nAr$ bedeuten, in der n 0 bis 4 ist und Ar einen Phenylring darstellt, gegebenenfalls substituiert mit 1 bis 3 Substituenten, ausgewählt aus $C_{1-4}$-Alkyl-, $C_{1-4}$-Alkoxy- oder $C_{1-4}$-Alkylthioresten, Halogenatomen, Cyano-, Amino-, Hydroxy-, Carbamoyl- oder Carboxylgruppen, $C_{1-4}$-Alkanoylresten oder Trifluormethylgruppen oder $R^2$ und $R^3$ zusammen mit dem Stickstoffatom, an das sie gebunden sind, einen gegebenenfalls ein oder mehrere weitere Heteroatome enthaltenden gesättigten oder ungesättigten Ring bilden, und

$R^4$ und $R^5$ gleich oder verschieden sind und jeweils ein Wasserstoffatom, einen $C_{1-4}$-Alkylrest oder einen Rest der Formel $-(CH_2)_nAr^1$ bedeuten, in der n 0 bis 4 ist und $Ar^1$ einen Phenylring darstellt, gegebenenfalls substituiert mit 1 bis 3 Substituenten, ausgewählt aus $C_{1-4}$-Alkyl-, $C_{1-4}$-Alkoxy-, oder $C_{1-4}$-Alkylthioresten, Halogenatomen, Cyano-, Amino-, Hydroxy-, Carbamoyl- oder Carboxylgruppen, $C_{1-4}$-Alkanoylresten oder Trifluormethylgruppen, oder $R^4$ und $R^5$ zusammen mit dem Stickstoffatom, an das sie gebunden sind, einen gegebenenfalls ein oder mehrere weitere Heteroatome enthaltenden gesättigten oder ungesättigten Ring bilden,
mit der Maßgabe, daß wenn jeder Rest $R^2$, $R^3$, $R^4$ und $R^5$ ein Wasserstoffatom bedeutet, $R^1$ nicht $CH_2NR^6R^7$ ist,
und pharmazeutisch verträgliche Salze davon.

2. Verbindung nach Anspruch 1, wobei einer der Reste $R^2$ und $R^3$ den Rest der Formel $(CH_2)_nAr$ bedeutet, in der n 0 ist und Ar eine Phenylgruppe bedeutet, und der andere Rest eine Methylgruppe darstellt, und einer der Reste $R^4$ und $R^5$ ein Wasserstoffatom darstellt und der andere einen Rest der Formel $(CH_2)_nAr^1$ bedeutet, in der n 0 ist und $Ar^1$ einen substituierten Phenylring darstellt.

3. Verbindung nach Anspruch 2, wobei $Ar^1$ mit einer Methylgruppe in 2-Stellung oder mit einer Methylgruppe in 2-Stellung und einem Fluoratom in der 4-Stellung substituiert ist.

4. Verbindung nach Anspruch 3, wobei $R^1$ eine Hydroxylgruppe bedeutet.

5. Verbindung nach Anspruch 3, wobei $R^1$ einen Hydroxy-$C_{1-4}$-alkoxyrest oder einen Rest der Formel $O(CH_2)_mNR^6R^7$ bedeutet, in der m, $R^6$ und $R^7$ wie für Struktur 1 in Anspruch 1 beschrieben definiert sind.

6. Verbindung nach Anspruch 1, nämlich
4-(N-Methylphenylamino)-2-(2-methylphenylamino)-8-hydroxychinazolin,
Bis-2,4-(N-methylphenylamino)-8-hydroxychinazolin-hydrochlorid
4-(N-Methylphenylamino)-2-[(2-methyl-4-fluorphenyl)amino]-8-hydroxychinazolinhydrobromid,
8-(3-Dimethylaminopropoxy)-2-[(2-methylphenyl)amino]-4-(N-methylphenylamino)chinazolin,
4-(N-Methylphenylamino)-2-[(2-methylphenyl)amino]-6-hydroxychinazolinhydrobromid,
4-(N-Methylphenylamino)-2-[(2-methyl-4-fluorphenyl)amino]-6-hydroxychinazolinhydrochlorid,
4-(N-Methylphenylamino)-2-[(2-methyl-4-fluorphenyl)amino]-6-(3-dimethylaminopropoxy)chinazolin,
4-(N-Methylphenylamino)-2-[(2-methyl-4-fluorphenyl)amino]-6-(3-morpholinopropoxy)-chinazolin,
4-(N-Methylphenylamino)-2-[(2-methyl-4-fluorphenyl)amino]-6-(2-dimethylaminoethoxy)chinazolin,
8-(Hydroxyethoxy)-4-(N-methylphenylamino)-2-[(2-methylphenyl)amino]chinazolin,
2-(2-Methyl-4-fluorphenylamino)-4-(N-methylphenylamino)-8-(3-morpholinopropoxy)chinazolin,
2-(2-Methyl-4-fluorphenylamino)-4-(N-methylphenylamino)-8-(3-dimethylaminopropoxy)chinazolin,
oder ein pharmazeutisch verträgliches Salz davon.

7. Arzneimittel, umfassend eine Verbindung nach einem der Ansprüche 1 bis 6 und einen pharmazeutischen Träger.

8. Verbindung nach einem der Ansprüche 1 bis 6 zur Verwendung als therapeutisches Mittel.

9. Verfahren zur Herstellung einer Verbindung nach Anspruch 1, umfassend:
(a) Umsetzung einer Verbindung der Struktur (II)

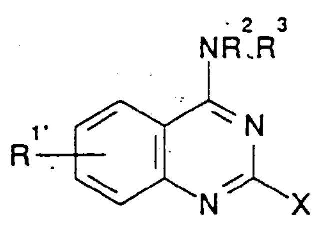

(II)

in der $R^2$ und $R^3$ wie für Struktur (I) beschrieben definiert sind, $R^{1'}$ einen gegebenenfalls geschützten Rest $R^1$ darstellt und X einen durch ein Amin ersetzbaren Rest bedeutet, mit einem Amin der Struktur $R^4R^5NH$, in der $R^4$ und $R^5$ wie für Struktur (I) beschrieben definiert sind;
(b) Umsetzung einer Verbindung der Struktur (III)

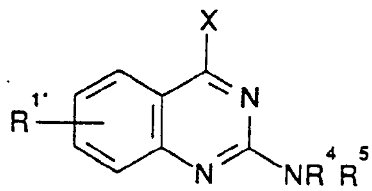

(III)

in der $R^{1'}$ wie für Struktur (II) beschrieben definiert ist, $R^4$ und $R^5$ wie für Struktur (I) beschrieben definiert sind, und X einen durch ein Amin ersetzbaren Rest bedeutet, mit einem Amin der Struktur $R^2R^3NH$, in der $R^2$ und $R^3$ wie für Struktur (I) beschrieben definiert sind; oder

(c) für Verbindungen der Struktur (I), in denen $NR^2R^3$ und $NR^4R^5$ gleich sind, Umsetzung einer Verbindung der Struktur (IV)

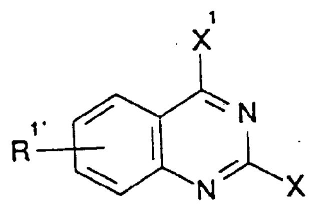

(IV)

in der $R^{1'}$ wie für Struktur (II) beschrieben definiert ist und X und $X^1$ beide durch ein Amin ersetzbare Reste bedeuten, mit einer Verbindung der Struktur $R^2R^3NH$ oder $R^4R^5NH$;

(d) für Verbindungen, in denen $R^1$ keine Hydroxylgruppe, oder keinen $C_{1-4}$-Alkyl-$NR^6R^7$- oder Hydroxy-$C_{1-4}$-alkylrest bedeutet, Alkylieren einer Verbindung der Struktur (V)

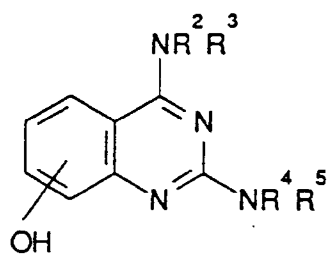

(V)

in der $R^2$ bis $R^5$ wie für Struktur (I) beschrieben definiert sind, und gegebenenfalls anschließend

- Entfernen von Schutzgruppen;
- Umwandeln eines Restes $R^{1'}$ in einen anderen Rest $R^1$;
- Herstellen eines pharmazeutisch verträglichen Salzes.

**10.** Verbindung der Struktur (II), (IV) oder (V) gemäß Anspruch 9 mit der Maßgabe, daß $R^{1'}$ in den Verbindungen der Formel (II) und (IV) keinen Alkoxyrest bedeutet.

## Revendications

**1.** Composé de structure (I) :

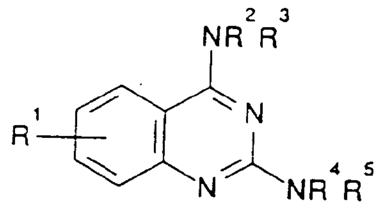

(I)

dans laquelle

$R^1$ est un groupe hydroxy, hydroxyalkyle en $C_{1-4}$ , hydroxyalcoxy en $C_{1-4}$ carboxyalcoxy en $C_{1-4}$ , alcoxy($C_{1-4}$)carbonylalcoxy en $C_{1-4}$ , alcoxy($C_{1-4}$)alcoxy en $C_{1-4}$ , $O(CH_2)_m$Het, alkyl($C_{1-4}$)-$NR^6R^7$ ou $O(CH_2)_mNR^6R^7$ dans lesquels m est 2 à 4 et $R^6$ et $R^7$ sont identiques ou différents et sont chacun un atome d'hydrogène, un groupe alkyle en $C_{1-4}$ , Het, ou -$(CH_2)_n$Ar dans lequel n est 0 à 4, Ar est un noyau phényle éventuellement substitué par 1 à 3 substituants choisis parmi un groupe alkyle en $C_{1-4}$ , alcoxy en $C_{1-4}$ , alkylthio en $C_{1-4}$ , un atome d'halogène, un groupe cyano, amino, hydroxy, carbamoyle, carboxy, alcanoyleen $C_{1-4}$ ou trifluorométhyle et Het est un noyau hétérocyclique à 5 ou 6 chaînons contenant un ou plusieurs hétéroatomes, ou $R^6$ et $R^7$ forment ensemble avec l'atome d'azote auquel ils sont fixés un noyau saturé ou insaturé contenant éventuellement un ou plusieurs autres hétéroatomes ;

$R^2$ et $R^3$ sont identiques ou différents et sont chacun un atome d'hydrogène, un groupe alkyle en $C_{1-4}$ , -$(CH_2)_n$Ar dans lequel n est 0 à 4 et Ar est un noyau phényle éventuellement substitué par 1 à 3 substituants choisis parmi un groupe alkyle en $C_{1-4}$ , alcoxy en $C_{1-4}$ , alkylthio en $C_{1-4}$ , un atome d'halogène, un groupe cyano, amino, hydroxy, carbamoyle, carboxy, alcanoyle en $C_{1-4}$ ou trifluorométhyle, ou $R^2$ et $R^3$ forment ensemble avec l'atome d'azote auquel ils sont fixés un noyau saturé ou insaturé contenant éventuellement un ou plusieurs autres hétéroatomes ; et

$R^4$ et $R^5$ sont identiques ou différents et sont chacun un atome d'hydrogène, un groupe alkyle en $C_{1-4}$ , -$(CH_2)_nAr^1$ dans lequel n est 0 à 4 et Ar est un noyau phényle éventuellement substitué par un à trois substituants choisis parmi un groupe alkyle en $C_{1-4}$ , alcoxy en $C_{1-4}$ , alkylthio en $C_{1-4}$ , un atome d'halogène, un groupe cyano, amino, hydroxy, carbamoyle, carboxy, alcanoyle en $C_{1-4}$ ou trifluorométhyle, ou bien $R^4$ et $R^5$ forment ensemble avec l'atome d'azote auquel ils sont fixés un noyau saturé ou insaturé contenant éventuellement un ou plusieurs hétéroatomes ;

à condition que lorsque $R^2$ , $R^3$, $R^4$ et $R^5$ sont tous un atome d'hydrogène, $R^1$ ne soit pas un groupe $CH_2NR^6R^7$, et sels de celui-ci acceptables du point de vue pharmaceutique.

**2.** Composé suivant la revendication 1, dans lequel l'un des $R^2$ et $R^3$ est un groupe $(CH_2)_n$Ar dans lequel n est 0 et Ar est un groupe phényle, et l'autre est un groupe méthyle, et l'un des $R^4$ et $R^5$ est un atome d'hydrogène et l'autre est un groupe $(CH_2)_nAr^1$ dans lequel n est 0 et $Ar^1$ est un noyau phényle substitué.

**3.** Composé suivant la revendication 2, dans lequel $Ar^1$ est substitué par un groupe méthyle en position 2, ou par un groupe méthyle en position 2 et un atome de fluor en position 4.

**4.** Composé suivant la revendication 3, dans lequel $R^1$ est un groupe hydroxy.

**5.** Composé suivant la revendication 3, dans lequel $R^1$ est un groupe hydroxy-alcoxy en $C_{1-4}$ ou $O(CH_2)_mNR^6R^7$ dans lequel m, $R^6$ et $R^7$ sont tels que décrits pour la structure (I) dans la revendication 1.

**6.** Composé suivant la revendication 1 qui est la :
4-(N-méthylphénylamino)-2-(2-méthylphénylamino)-8-hydroxyquinazoline,
le chlorhydrate de bis-2,4-(N-méthylphénylamino)-8-hydroxyquinazoline,
le chlorhydrate de 4-(N-méthylphénylamino)-2-[(2-méthyl-4-fluorophényl)amino]-8-hydroxyquinazoline,
la 8-(3-diméthylaminopropoxy)-2-[(2-méthylphényl)amino]-4-(N-méthylphénylamino)quinazoline,

le bromhydrate de 4-(N-méthylphénylamino)-2-[(2-méthylphényl)amino]-6-hydroxyquinazoline,
le chlorhydrate de 4-(N-méthylphénylamino)-2-[(2-méthyl-4-fluorophényl)amino]-6-hydroxyquinazoline,
la 4-(N-méthylphénylamino)-2-[(2-méthyl-4-fluorophényl)amino]-6-(3-diméthylaminopropoxy)quinazoline,
la 4-(N-méthylphénylamino)-2-[(2-méthyl-4-fluorophényl)amino]-6-(3-morpholinopropoxy)quinazoline,
la 4-(N-méthylphénylamino)-2-[(2-méthyl-4-fluorophényl)amino]-6-(2-diméthylaminoéthoxy)quinazoline,
la 8-(hydroxyéthoxy)-4-(N-méthylphénylamino)-2-((2-méthylphényl)amino]quinazoline,
la 2-(2-méthyl-4-fluorophénylamino)-4-(N-méthylphénylamino)8-(3-morpholinopropoxy)quinazoline,
la 2-(2-méthyl-4-fluorophénylamino)-4-(N-méthylphénylamino)8-(3-diméthylaminopropoxy)quinazoline,
ou un sel de ceux-ci acceptable du point de vue pharmaceutique.

7. Composition pharmaceutique comprenant un composé suivant l'une quelconque des revendications 1 à 6 et un support pharmaceutique.

8. Composé suivant l'une quelconque des revendications 1 à 6 utile en tant qu'agent thérapeutique.

9. Procédé pour la préparation d'un composé suivant la revendication 1, qui comprend :
(a) la réaction d'un composé de structure (II) :

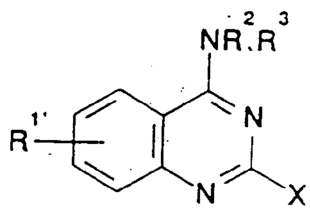

(II)

dans laquelle $R^2$ et $R^3$ sont tels que définis pour la structue (I), $R^{1'}$ est un groupe $R^1$ éventuellement protégé et X est un groupe pouvant être déplacé par une amine, avec une amine de structure $R^4R^5NH$ dans laquelle $R^4$ et $R^5$ sont tels que décrits pour la structure (I) ;
(b) la réaction d'un composé de structure (III) :

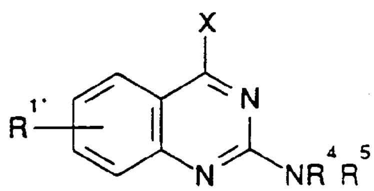

(III)

dans laquelle $R^{1'}$ est tel que décrit pour la structure (II), $R^4$ et $R^5$ sont tels que décrits pour la structure (I) et X est un groupe pouvant être déplacé par une amine, avec une amine de structure $R^2R^3NH$ dans laquelle $R^2$ et $R^3$ sont tels que décrits pour la structure (I) ; ou
(c) pour des composés de structure (I) dans laquelle $NR^2R^3$ et $NR^4R^5$ sont identiques, la réaction d'un composé de structure (IV) :

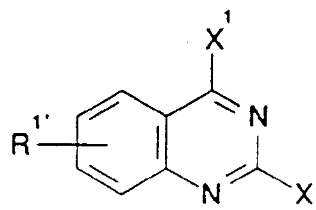

(IV)

dans laquelle $R^{1'}$ est tel que décrit pour la structure (II) et X et $X^1$ sont tous deux des groupes pouvant être déplacés par une amine, avec un composé de structure $R^2R^3NH$ ou $R^4R^5NH$ .
(d) pour des composés dans lesquels $R^1$ est autre qu'un groupe hydroxy, alkyl($C_{1-4}$)$NR^6R^7$ ou hydroxy-alkyle en $C_{1-4}$ , l'alkylation d'un composé de sturcture (V) :

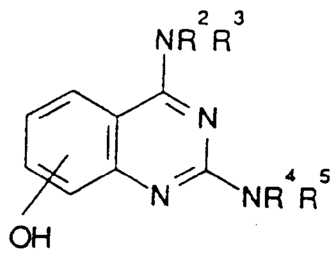

(V)

dans laquelle $R^1$ à $R^5$ sont tels que décrits pour la structure (I) ; et éventuellemnt ensuite,
- ° l'élimination de quelconques groupes protecteurs,
- ° la conversion d'un groupe $R^{1'}$ en un autre groupe $R^1$ ;
- ° la formation d'un sel acceptable du point de vue pharmaceutique.

**10.** Composé de structure (II), (IV) ou (V) tel que décrit dans la revendication 9, à condition que $R^{1'}$ ne soit pas un groupe alcoxy dans les composés de formules (II) et (IV).